Europäisches Patentamt

European Patent Office

Office européen des brevets

(11) Publication number: **0 369 786**
**A1**

# EUROPEAN PATENT APPLICATION

(21) Application number: 89311881.0

(22) Date of filing: 16.11.89

(51) Int. Cl.⁵: **A61F 5/00**

(30) Priority: 16.11.88 AU 1498/88

(43) Date of publication of application:
**23.05.90 Bulletin 90/21**

(84) Designated Contracting States:
**AT BE CH DE ES FR GB GR IT LI LU NL SE**

(71) Applicant: **UNISEARCH LIMITED**
**Unisearch House 221-227 Anzac Parade**
**Kensington New South Wales, 2033(AU)**

(72) Inventor: **Frost, Richard**
**10 Lansdown Street**
**Eastwood New South Wales 2122(AU)**

(74) Representative: **Abbie, Andrew Kenneth et al**
**R.G.C. Jenkins & Co. 26 Caxton Street**
**London SW1H 0RJ(GB)**

(54) **Friction self-locking joint for whole-leg calipers.**

(57) A self-locking knee joint for a whole-leg caliper,
the joint has a thigh part and a calf part connected
by a pivot assembly. The joint also includes locking
surfaces which are activated by the user applying
weight to the thigh part, which locking surfaces fix
the thigh part to the calf part during a gait swing
phase, but release the calf part during a gait stance
phase.

FIG. 2

## FRICTION SELF-LOCKING JOINT FOR WHOLE-LEG CALIPERS

The present invention relates to a knee joint for whole-leg calipers to support disabled people.

More particularly, but not exclusively, the present invention relates to a friction-locking knee joint which may form a module to be incorporated in an overall caliper assembly, or to form an integral part of a caliper by being integrally formed with the thigh and calf bars.

Previously known knee joints have merely consisted of a pivot. A means was provided to prevent pivotting of the knee joint during a normal walking operation. Because the knee joint was effectively rendered rigid, walking was difficult and required the user to raise each leg alternately or to cause each leg to swing laterally outward to clear the ground.

If the user was to sit, the knee joint needed to be operated so that a pivotting action could take place.

A still further disadvantage was failure of the pivot joint to support the user, if the user transferred their weight forward of the knee joint. This problem was exacerbated by the pivot not being at the center of the knee.

Australian Patent Specification 73268/81, describes a knee stabilizing device which acts as a brace for the knee. The brace has upper and lower portions, with a central portion to be positioned adjacent the knee. The central portion cooperates with the upper and lower portions to limit the angular deflection of the knee. This limited movement would make walking difficult.

Australian Patent Specification 88214/82 describes an adjustable splint. The splint has a knee joint which is basically a pivot connection. The pivot connection does not control movement of the knee.

Australian Patent Specification 85392/82 describes a knee brace to support the knee. The knee brace includes upper and lower parts which are pivotally connected. No consideration is given to controlling movement of the knee.

Australian Patent Specification 29167/84 describes a knee brace similar to the previously described knee brace. The pivot connection merely prevents twisting of the knee and does not provide any control over angular deflection of the upper and lower parts of the brace.

Australian Patent Specification 75427/87 also describes a knee brace, with the pivot joint providing unhindered pivotting of the upper and lower portions.

Australian Patent Specification 59004/86 is similar to the previously described knee braces however the pivot joint is provided by a pivotted link, which does not control knee pivotting.

Australian Patent Specification 89513/82 is similar to the previously discussed knee brace except that there is some restraint in respect of knee movement.

Australian Patent Specification 28950/87 describes a knee brace with a pivot joint providing some lateral movement in the pivot. Again there is no restriction in respect of knee pivotting.

Australian Patent Specification 29908/71 describes a knee brace which permits pivotting about a single knee axis. There is no restriction to pivotting about this axis.

Australian Patent Specification 80971/82 describes a knee brace with upper and lower portions connected by a pivot which limits the angular deflection of the knee. Within this angular deflection, there is no restriction in respect of knee movement.

Australian Patent Specification 12235/88 describes a knee brace restricting movement about a single transverse axis. Apart from this restriction, the knee can pivot freely.

Australian Patent Specification 14519/83 is similar to the above discussed brace in that movement is restricted about one axis. Within specific ranges, there is no restriction for pivotting movement about that axis.

Australian Patent Specification 72216/74 describes a locking knee joint for a caliper. The knee joint may be locked with the upper and lower parts vertically aligned so that the user can walk with their leg restrained in a generally rigid straight configuration. The knee joint can be released by operation of a lever so that the user may bend at the knee to sit.

All of the above discussed published specifications do not permit the upper and lower parts of the knee joint to be alternatively restrained and released to facilitate walking.

It is the object of the present invention to overcome or substantially ameliorate the above disadvantages.

There is disclosed herein a knee joint comprising:

a thigh part;

a calf part:

pivot means pivotally coupling the thigh part and calf part for limited angular deflection therebetween about a horizontal transverse axis; and

locking means to lock the thigh part to the calf part during a gait swinging phase and to selectively release the thigh part from the calf part for pivotting movement therebetween about the transverse axis during the gait stance phase.

A preferred form of the present invention will now be described by way of example with reference to the accompanying drawings, wherein:

Figure 1 is a schematic side elevation of a knee joint for whole-leg calipers; and

Figure 2 is a schematic side elevation of a modification of the knee joint of Figure 1.

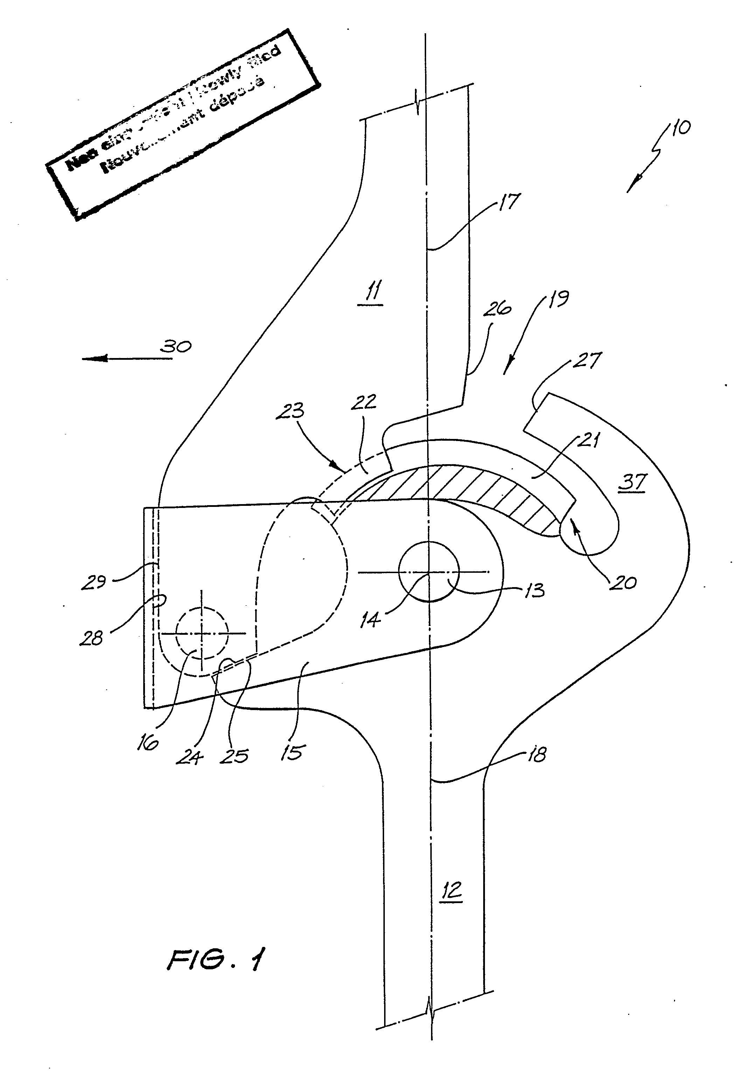

In figure 1 of the accompanying drawings there is schematically depicted a knee joint 10 including a thigh portion 11 and a calf portion 12. The thigh portion 11 may be formed integrally with or adapted to be attached to the thigh bar or bars of a hole-leg caliper. The calf portion 12 may also be formed integral with or be adapted to be attached to the calf bar or bars of the caliper.

The portions 11 and 12 are pivotally attached by means of a pin 13 which pivotally couples a link 15 with the calf portion 12. A further pin 16 pivotally couples the link 15 with the thigh portion 11. The thigh portion 11 is limited in respect of its pivotting movement relative to the link 15 about the pin 16 and accordingly defective pivotting movement of the thigh portion 11 is about the transverse horizontal axis 14 defined by the pin 13.

In this particular embodiment, the longitudinal axes 17 and 18 of the portions 11 and 12 pass through the transverse axis 14.

The knee joint 10 is provided with a locking means 19 which selectively prevents pivotting movement between the portions 11 and 12, when the user transferrs weight to the portion 11 and therefore the portion 12. The locking means 19 includes a first friction member 20 which has a pair of friction surfaces 21 which extend angularly about the axis 14 at a generally constant radius. The surfaces 21 converge radially outwardly so that in transverse cross section, the surfaces 21 form an inverted "V" shape. The locking member 20 is attached to the calf portion 12.

The locking means 19 also includes a second locking member 23 which engages the locking member 20. The locking member 23 is fixed to the thigh portion 11, and provides a pair of friction surfaces 22. The friction surfaces 22 also extend angularly about the axis 14 and converge radially outwardly so as to matingly engage the surfaces 20.

When weight is applied to the thigh portion 11, the friction member 23 frictionally engages the friction member 20 to thereby transfer the weight to the portion 12. The frictional engagement of the surfaces 21 and 22 also prevents relative rotation between the portions 11 and 12 about the axis 14. It should further be appreciated that friction or contact is enhanced by having the surfaces 21 and 22 inclined by acute angles to the axes 17 and 18.

The angular deflection of the thigh portion 11 relative to the calf portion 12 about the axis 14 is defined by two cooperating pairs of stop surfaces 24 and 25, and 26 and 27. With the surfaces 24 and 25 engaged, the axes 17 and 18 are generally vertically aligned.

The link 15 is provided with a forward abutment surface 28 which engages a forward surface 29 of the thigh portion 11 to limit forward tilting of the thigh portion 11 about the pin 16 relative to the link 15. This movement is limited to relatively small deflections and is merely provided to reduce frictional contact between the surfaces 21 and 22 to thereby permit pivotting movement between the portions 11 and 12 about the axis 14.

The knee joint 10, when forming part of a hole-leg caliper assembly, is intended for normal leg movement in a forward direction indicated by the arrow 30. Initially, weight is removed from the thigh portion 11 thereby disengaging the surfaces 22 from the surfaces 21. This then permits relative rotation between the portions 11 and 12 about the axis 14. The user then pivots the thigh portion 11 forward taking with it the thigh portion 11, which at the same time, pivots about the axis 14. This enables the user's foot to clear the ground surface. When weight is reapplied to the thigh portion 11, independent of the angular deflection of the portion 11 relative to the portion 12, the surfaces 22 frictionally engage the surfaces 21 locking the portions 11 and 12 together. This then permits rearward pivotting of the portion 11 and the portion 12. When it is again desired to move the portion 11 pivotally forward, the weight is removed from the portion 11 thereby permitting pivotting between the portions 11 and 12.

The stop surface 27 is formed on a projection 37. It has been found that the stop surface 27 is only required during initial use of the knee joint 10, when the user is learning how to use the knee joint 10. Once the user can competently use the knee joint 10, the projection 37 may be removed. Accordingly, the above discussed knee joint 10 may be modified by having the projection 37 removably attached to the portion 12.

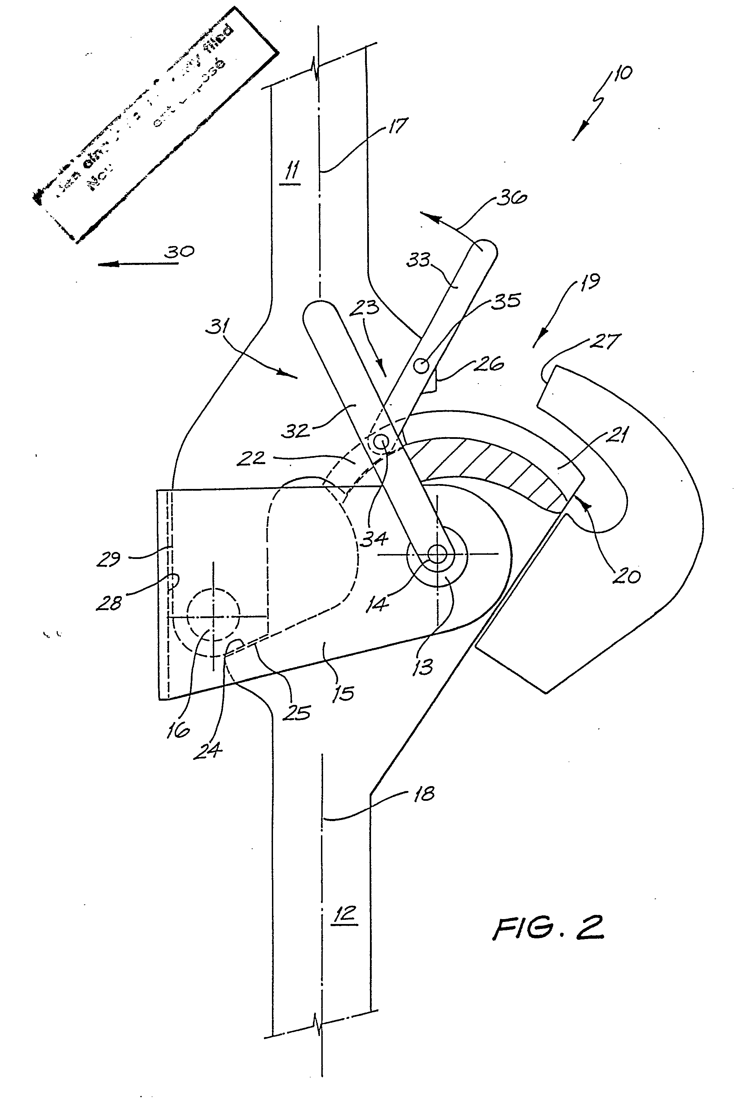

In the preferred form shown in Figure 2, the knee joint 10 is provided with a toggle mechanism 31. The remaining portions of the embodiment of Figure 2 have been allocated the same numerals as the corresponding portions of the embodiment of Figure 1.

The knee joint of Figure 2 is provided with the toggle assembly 31 to enable the user to manually displace the surfaces 22 from the surfaces 21. This function is desirable in order to facilitate bending of the knee joint 10 when the user wishes to sit.

The toggle assembly 31 includes a pair of links 32 and 33, with the link 33 being pivotally attached at one extremity to the link 32 by means of a pin 34. The link 33 is also pivotally attached to the portion 11 by means of a pin 35. One extremity of the link 32 is pivotally attached at the axis 14 to the portion 12.

In use of the toggle assembly 31, the user can disengage the surfaces 22 from the surfaces 21 by pivotting the link 33 in the direction of the arrow 36. This moves the surfaces 22 from the surfaces 21 and thereby permits pivotting of the portion 11 relative to the portion 12.

In the embodiment of Figure 2, the axes 17 and 18 are vertically aligned when the surfaces 24 and 25 are engaged, however in this embodiment the axes 17 and 18 are displaced forward of the axis 14 and do not pass therethrough.

The essential function of the above described joint 10 is that when weight is applied to it, it will allow the thigh and calf sections of the caliper to rotate freely relative to each other in the plane of walking, and when weight is applied to the joint 10, the joint 10 will lock against such relative rotation in whatever angular relationship exists between the thigh and calf sections at the time of such load application, provided such angular relationship is within certain limits.

The effect is thereby produced whereby the disabled user can, by suitable body movements, take weight off a leg supported by a caliper containing the knee joint 10, and by way of body movements cause the leg to move forward through the swing phase of the gait cycle without the necessity for circumduction, as the unlocking of the knee joint 10 will allow the calf to initially swing back relative to the thigh to allow the foot to avoid striking the floor or ground surface as it moves forward. Such swing phase of the gait is terminated by the calf swinging forward relative to the thigh, and by heel strike. At or close to the time of heel strike, weight is reapplied to the caliper by the user, thereby relocking the knee joint 10 at or close to the fully extended position of the knee joint 10 so that the caliper can provide weight-bearing and support throughout the insuing stance phase of the gait cycle.

## Claims

1. A knee joint comprising:
a thigh part;
a calf part;
pivot means pivotally coupling the thigh part and calf part for limited angular deflection therebetween about a horizontal transverse axis; and
locking means to lock the thigh part to the calf part during a gait swinging phase and to selectively release the thigh part from the calf part for pivotting movement therebetween about the transverse axis during the gait stance phase.

2. The knee joint of claim 1 wherein said locking means is activated to lock said thigh part to said calf part by a user applying a weight to said thigh part.

3. The knee joint of claim 2 wherein said locking means include a first locking member affixed to said calf part, and a second locking member affixed to said thigh part, which locking members frictionally engage to lock the thigh part to the calf part.

4. The knee joint of claim 3 wherein each locking member includes at least one friction surface extending angularly about said transverse axis, which frictional surfaces engage to lock said thigh part to said calf part.

5. The knee joint of claim 4 wherein each locking member has two frictional surfaces each extending angularly about said axis, with the frictional surfaces of each locking member converging radially outwardly, with the friction surfaces of said first locking member being adapted to matingly engage the friction surfaces of said second locking member.

6. The knee joint of claim 1 wherein said pivot means includes a link pivotally attached to said calf part and pivotally attached to said thigh part so as to pivot relative thereto about two spaced transverse axes.

7. The knee joint of claim 6 futher including stop surfaces to limited the angular deflection of the thigh part relative to the calf part.

8. The knee joint of claim 7 further including abutment means to limit pivotting of the thigh part relative to the link, but defining sufficient angular movement therebetween to release said thigh part from being locked to said calf part.

9. The knee joint of claim 8 further including manually operable means to cause pivotting between said thigh part and said link to release said thigh part from said calf part.

10. The knee joint of claim 9 wherein said manually operable means is a toggle including a first toggle link pivotally attached to said calf part, and a second toggle link pivotally attached to said thigh part and said first toggle link so that upon application of a force to said second toggle link, said thigh part is released from said calf part.

10

17

11

30

26    19

23    22    27

21

37

29

13

14

20

28

16    24    25    15

18

12

FIG. 1

Neu eingereicht / Newly filed
Nouvellement déposé

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-3 826 251 (ROSS)<br>* Abstract; figures * | 1,2 | A 61 F 5/00 |
| A | US-A-4 252 111 (CHAO et al.)<br>* Abstract * | 1 | |
| D,A | AU-A- 480 049 (CLARKE)<br>* Claim 1; figures * | 8,9 | |

TECHNICAL FIELDS
SEARCHED (Int. Cl.5)

A 61 F

The present search report has been drawn up for all claims

| Place of search | Date of completion of the search | Examiner |
|---|---|---|
| THE HAGUE | 12-02-1990 | SANCHEZ Y SANCHEZ J. |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P0401)